# FASCICULE DE BREVET EUROPEEN

(11) **EP 3 017 126 B1**
(45) Date de publication et mention de la délivrance du brevet: **02.08.2017**
(21) Numéro de dépôt: 14736769.2
(22) Date de dépôt: 04.07.2014
(51) Int. Cl.: E04H 1/12, A61G 10/00, A61M 21/00, E04B 1/344, E04H 3/08

(54) **CABINE PLIABLE D'ISOLATION SENSORIELLE**
FALTBARE KABINE FÜR SENSORISCHE ISOLIERUNG
FOLDABLE SENSORY DEPRIVATION BOOTH

(30) Priorité: 05.07.2013 FR 1356671
(43) Date de publication de la demande: 11.05.2016
(73) Titulaire: Reperes, 75011 Paris (FR)
(72) Inventeur: SAUNIER, Franck, F-77300 Fontainebleau (FR)
(74) Mandataire: Hirsch & Associés
(86) Numéro de dépôt international: PCT/EP2014/064328
(87) Numéro de publication internationale: WO 2015/001086

(56) Documents cités:
- WO-A2-2012/031306
- FR-A1- 2 705 033
- US-A1- 2004 074 212

## Description

La présente invention concerne une cabine pliable d'isolation sensorielle.

Dans le domaine de l'analyse sensorielle, il est connu de disposer des participants à une étude dans des locaux d'analyse sensorielle aménagés au sein de laboratoires dont l'environnement intérieur est contrôlé. En particulier, un contrôle de l'hygrométrie et de la température et une filtration de l'air sont généralement réalisés dans de tels laboratoires.

Le problème majeur de ces laboratoires est que leur installation est coûteuse et complexe de sorte que le nombre et l'accessibilité des laboratoires sont limités.

De plus, l'environnement peut différer d'un laboratoire à un autre de sorte que les résultats des analyses sensorielles souffrent d'un défaut de reproductibilité et peuvent alors être faussés.

Il existe donc un besoin pour des analyses sensorielles dans un environnement plus stable et plus accessible à un large public. US 2004/0074212, FR 2 705 033 et WO 2012/031306 divulguent des cabines d'isolation sensorielles qui font partie de l'état de la téchnique pertinent. A cette fin, la présente invention propose une cabine d'isolation sensorielle pliable entre :
- une position repliée de transport et
- une position dépliée d'utilisation formant un volume fermé d'accueil d'un utilisateur pour réaliser une analyse sensorielle ;
la cabine comportant :
- des parois, chaque paroi étant étanche à l'air ou adaptée à filtrer la circulation d'air traversant la paroi ;
- des articulations et des dispositifs de fermeture étanches à l'air, disposés à la jointure des parois,
les articulations et les dispositifs de fermetures fermées reliant les parois de manière étanche à l'air dans la position dépliée d'utilisation de la cabine,
les articulations repliées autorisant la superposition des parois les unes sur les autres dans la position de transport de la cabine, caractérisée en ce que les parois comprennent une première et une deuxième parois réalisées en un même matériau commun, la première et la deuxième parois étant reliées entre elles de manière étanche par le matériau commun qui est aminci localement pour former une des articulations. Suivant des modes de réalisation préférés, l'invention comprend une ou plusieurs des caractéristiques suivantes :
- les dimensions et le nombre de parois sont choisis de sorte que le volume fermé d'accueil dans la position d'utilisation soit supérieur ou égal à 1,9*1*1 = 1.5 m3 de préférence supérieur ou égale à 1,9m3 et que le volume d'une enveloppe externe parallélépipédique de la cabine dans la position de transport soit inférieur ou égal à 0.5 m3 de préférence inférieur ou égal à 1*1.9*0.1 = 0.2m3.
- l'enveloppe externe parallélépipédique de la cabine dans la position de transport présente une longueur inférieure ou égale à 2.5m, de préférence inférieure ou égale à 2m, une largeur inférieure ou égale à 1.5m, de préférence inférieure ou égale à 1.1m et une épaisseur inférieure ou égale à 0.3m, de préférence inférieure ou égale à 0.1m.
- le poids de la cabine étant inférieur ou égale à 25Kg, de préférence inférieur ou égale à 23Kg.
- les parois sont réalisées en matériau alvéolaire, de préférence en PVC alvéolaire.
- les parois comprennent une paroi formant une porte ou une trappe d'accès au volume fermé d'accueil, la porte ou la trappe comportant un dispositif de fermeture étanche à l'air.
- les parois comprennent une paroi formant une porte ou une trappe d'accès au volume fermé d'accueil, la porte ou la trappe comportant un dispositif de fermeture étanche à l'air qui est à fermeture magnétique.
- les parois comportent :
   * au moins une paroi horizontale comprenant une paroi supérieure et de préférence une paroi inférieure ;
   * des parois latérales ;
   les dispositifs de fermeture étanche de la cabine comprenant, à la jointure de l'au moins une paroi horizontale et des parois latérales dans la position dépliée d'utilisation de la cabine, un dispositif de fermeture étanche comportant au moins un parmi un joint élastomère et une gorge, l'étanchéité à la jointure de l'au moins une paroi horizontale et des parois latérales étant réalisée par la compression du dispositif de fermeture étanche entre les parois latérales et l'au moins une paroi horizontale sous l'effet du poids des parois.
- les parois comportent une paroi munie d'une cartouche de filtration adaptée à filtrer l'air circulant au travers de la paroi.

L'invention propose aussi un système d'analyse sensorielle comprenant :
- une ventilation mécanique contrôlée ;
- la cabine précédente comportant une ouverture adaptée à être reliée de manière étanche à la ventilation mécanique contrôlée pour le renouvellement de l'air du volume fermé d'accueil dans la position d'utilisation.

D'autres caractéristiques et avantages de l'invention apparaîtront à la lecture de la description qui suit d'un mode de réalisation préféré de l'invention, donnée à titre d'exemple et en référence aux dessins annexés.
La figure 1 montre une vue en perspective d'une cabine d'isolation sensorielle dans un une position d'utilisation et selon un mode de réalisation.
La figure 2 montre une vue partielle en perspective de la cabine de la figure 1 et dans la position de transport.
La figure 3 montre une vue de dessus de la cabine de la figure 1.
Les figures 4 et 5 montrent une vue agrandie d'un mode de réalisation d'une articulation reliant deux parois adjacentes de la cabine respectivement en position plié et en position dépliée
La figure 6 montre les jointures entre la porte de la cabine et les parois adjacentes de la porte de la cabine.
La figure 7 montre une vue en perspective de l'arrière de la cabine de la figure 1.
La figure 8 montre une vue en perspective d'un mode de réalisation de la paroi inférieure de la cabine.
La figure 9 montre une vue agrandie de la figure 8 au niveau d'une jointure de la paroi inférieure et d'une des parois latérales de la cabine.
La figure 10 montre une vue en perspective d'un mode de réalisation d'une paroi supérieure de la cabine.
Les figures 11 et 12 montrent des vues agrandies de la figure 10 aux intersections entre des jointures de parois de la cabine.
La figure 13 montre une vue en perspective d'un autre mode de réalisation d'une paroi supérieure de la cabine.
La figure 14 montre une vue agrandie de la figure 13 à l'intersection de jointures de parois.
Les figures 15 et 16 montrent une tablette 70 avec des équerres respectivement mise à plat et en position perpendiculaire.
La figure 17 montre une vue agrandie d'une équerre de la tablette des figures 15 et 16 reçue dans un support d'une paroi de la cabine.
La figure 18 montre un pain de mousse sous la forme d'une cale trapézoïdale pour le passage de raccordement entre l'intérieur et l'extérieur de la cabine.
La figure 19 montre une vue agrandie de la cale de la figure 18.

Il est proposé une cabine d'isolation sensorielle. L'isolation sensorielle visée par la cabine permet la réalisation d'analyses sensorielles correspondant par exemple à des analyses visuelles, olfactives, organoleptiques ou encore tactiles d'un produit.

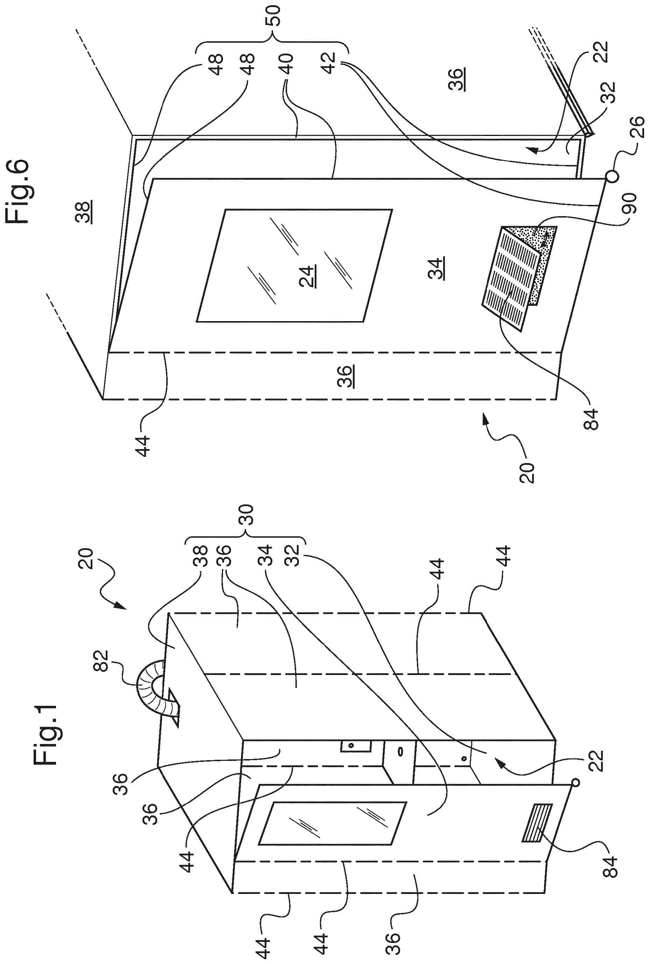

La figure 1 montre la cabine 20 selon un mode de réalisation et dans une position d'utilisation. Cette cabine 20 forme un volume 22 d'accueil d'un utilisateur (non représenté) pour réaliser une analyse sensorielle. La cabine 20 d'isolation sensorielle proposée est ainsi par exemple de dimensions adaptées pour recevoir une personne humaine, par exemple de taille adulte, le volume d'accueil 22 pouvant être supérieur ou égal à 1.5 m³, et de préférence supérieur ou égal à 1,9m³.

Tel qu'illustré, dans la position d'utilisation, le volume d'accueil 22 de la cabine peut être fermé, ici à l'aide d'une porte 34. Le caractère fermé, c'est-à-dire isolé, du volume d'accueil 22 formé par la cabine 20 dans la position d'utilisation permet un contrôle des conditions de l'analyse sensorielle à l'intérieur de la cabine 20. Dans le cas notamment d'analyses visuelles, la fermeture du volume 22 permet le contrôle de la luminosité. Le contrôle d'un ensemble d'autres paramètres influant sur la perception sensorielle de l'utilisateur peut en outre être envisagé : maintien de la température, de l'hygrométrie, insonorisation par rapport à l'environnement extérieur. Dans le cas particulièrement plus visé d'analyses olfactives ou organoleptiques, la fermeture du volume 22 permet un contrôle de la qualité de l'air dans la cabine 20 en limitant l'entrée d'odeur parasite de l'extérieur vers l'intérieur de la cabine 20. La cabine 20 proposée dans la position d'utilisation avec son volume fermé d'accueil 22 permet ainsi de diminuer les perturbations extérieures, notamment olfactives, pour l'utilisateur lors d'une analyse sensorielle effectuée dans la cabine 20.

Dans ce même but de limiter les perturbations extérieures tel que l'entrée d'odeur parasite de l'extérieur vers l'intérieur, les parois 30 composant la cabine 20 sont étanches à l'air. La porte 34 fait partie de l'ensemble 30 des parois 32, 36 et 38 composant la cabine sensorielle 20. Les parois 32 et 38 ici représentés sont des parois horizontales dans la position d'utilisation, la paroi 32 étant la paroi inférieure de la cabine 20, par exemple un tapis, et la paroi 38 étant la paroi supérieure de la cabine 20, autrement dénommé toit. Les parois 36 correspondent aux parois verticales formant la cabine 20 y compris la porte 34 qui est une variante particulière d'une des parois verticales 36 de la cabine 20. Dans la suite de ce document, l'expression « paroi 30 » désigne de manière indifférenciée n'importe quelle paroi de l'ensemble de parois composant la cabine 20 alors que les signes de référence, 32, 34, 36, et 38 désignent de manière particulière chaque type de paroi ci-dessus décrit.

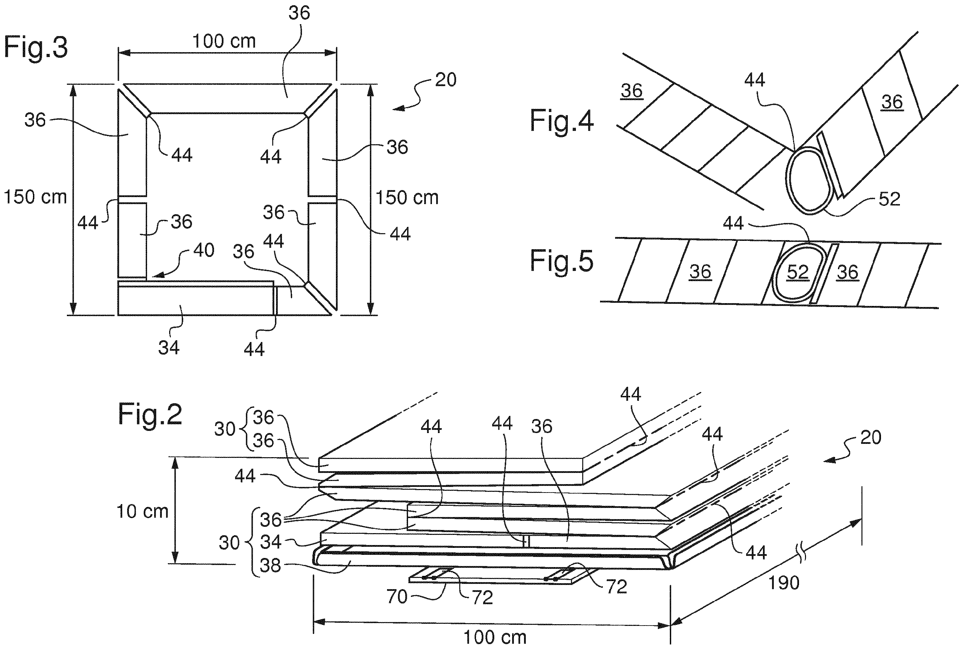

En plus d'une position d'utilisation, la cabine 20 proposée présente une position de transport. La figure 2 montre une vue partielle en perspective de la cabine 20 selon le mode de réalisation de la figure 1 et dans la position de transport. Telles qu'illustrées, les parois 34, 36 et 38 de la cabine 20 sont superposées les unes sur les autres dans la position de transport. La position de transport de la cabine 20 présente un faible encombrement facilitant le transport de la cabine. La figure 2 représente en cm, les dimensions d'une enveloppe externe parallélépipédique de la cabine 20 dans la position de transport. Selon le mode de réalisation illustré, l'enveloppe externe parallélépipédique présente une longueur de 190 cm, une largeur de 100 ou 106cm et une épaisseur de 10 cm. Toutefois d'autres dimensions peuvent être envisagées pour la compacité dans la position de transport, telle que :
- une longueur inférieure ou égale à 2m voir inférieure ou égale à 2,5 m ;
- une largeur inférieure ou égale à 1,1m voir inférieure ou égale à 1,5 m ;
- une épaisseur inférieure ou égale à 0,15m voir inférieure ou égale à 0,3m.

Pour réduire encore l'encombrement de la cabine 20 dans la position de transport, la cabine 20 peut être dépourvue de la paroi inférieure 32 (non illustré en figure 2), le sol d'un local pouvant suffire à fermer le volume d'accueil 22 de la cabine 20 dans la position d'utilisation. De façon alternative, la paroi inférieure 32 peut être formée par un tapis souple, facile à enrouler et qui peut être disposé dans le volume externe parallélépipédique illustré en figure 2. Selon les dimensions et le nombre de parois 30 de la cabine 20, le volume de l'enveloppe externe parallélépipédique peut ainsi être inférieur ou égal à 0,5 m³ ou même être inférieur ou égal à 0,2 m3 conformément aux dimensions illustrées en figure 2.

Pour permettre le passage réversible de la position d'utilisation à la position de transport, la cabine 20 proposée est une cabine pliable. La position d'utilisation illustrée en figure 1 correspond à la position dépliée, alors que la position de transport illustrée en figure 2 correspond à la position repliée. La cabine 20 proposée comprend des articulations aux jointures entre certaines des parois 30 permettant le pliage et le dépliage de la cabine 20. Dans ce document, une jointure entre deux parois 30 correspond à l'intersection formée entre les deux parois 30 adjacentes dans la position d'utilisation de la cabine 20. Une jointure entre deux parois 30 dans la position d'utilisation de la cabine 20 se sépare dans la position de transport en une lèvre sur l'une des parois 30 et une lèvre sur l'autre des parois 30. Dans ce document, les deux lèvres d'une même jointure portent le même signe de référence que la jointure elle-même.

La figure 3 montre une vue de dessus de la cabine 20, en l'absence de la paroi supérieure 38. Telles qu'illustrées, les parois 36 et la porte 34 sont reliées deux à deux par des articulations disposées à chaque jointure 44 entre deux parois 34 ou 36 adjacentes. Les articulations sont figurées dans cette demande par des traits mixtes. Dans la position d'utilisation de la cabine 20, les articulations peuvent être pliées ou dépliées selon les parois 34 ou 36 qu'elles relient. L'articulation à la jointure entre la porte 34 et la paroi 36 adjacente peut ainsi être dépliée lorsque la porte 34 est fermée, alors que l'articulation à la jointure entre cette même paroi 36 et la paroi 36 adjacente est pliée dans la position d'utilisation de la cabine.

Les figures 4 et 5 montrent une vue agrandie d'un mode de réalisation d'une articulation reliant deux parois adjacentes respectivement en position plié et en position dépliée. Selon le mode de réalisation illustré, les deux parois 36 sont réalisées en un même matériau commun, ici un matériau alvéolaire, et ces deux parois 36 sont reliées entre elles par ce matériau commun qui forme l'articulation à la jointure 44. L'articulation est alors formée par un amincissement local du matériau commun entre les deux parois 36. Tel qu'illustré en figures 4 et 5, l'amincissement local du matériau commun correspond à une découpe du matériau alvéolaire de sorte que seule une des peaux du matériau alvéolaire assure la continuité de matière entre les deux parois 36. Une telle continuité de matière entre les deux parois en matériau étanche à l'air assure une étanchéité de l'articulation. L'articulation peut en outre comprendre un joint creux 52 d'isolation phonique et/ou thermique entre les deux lèvres de la jointure 44. D'autres modes de réalisation d'articulation étanche peuvent être envisagés tel que des charnières rapportées, par exemple adhésives, et recouverte d'un matériau étanche le long de la jointure entre les parois 30. Toutefois, le mode de réalisation illustré en figures 4 et 5 est préféré par sa simplicité et sa légèreté.

Au niveau des jointures dépourvues d'articulations étanches, la cabine 20 comporte des dispositifs de fermeture étanches à l'air pour fermer le volume 22 dans la position d'utilisation de la cabine 20.

En d'autres termes, les jointures entre les parois adjacentes 30 de la cabine 20 sont :
- soit inamovibles comme dans le cas des articulations étanches ;
- soit amovibles comme dans le cas des dispositifs de fermeture étanches.

Dans tous les modes de réalisation de jointures proposées, les parois 30 sont mobiles les unes par rapport aux autres au niveau de leur jointure, ce qui permet le passage de la cabine 20 de la position d'utilisation à la position de transport, où les parois 30 sont superposées les unes sur les autres, et inversement.

Le caractère amovible du dispositif de fermeture étanche permet de maintenir l'étanchéité du volume fermé d'accueil 22 tout en permettant une désolidarisation locale et facile des parois 30. Cette désolidarisation peut permettre dans la position d'utilisation d'accéder rapidement à l'intérieur de la cabine 20 sans démonter la cabine 20. Cette désolidarisation des parois 30 permet encore, dans la position de transport, de contribuer à la compacité de la cabine 20. Telle qu'illustré en figure 2, la paroi supérieure 38 peut ainsi être complétement désolidarisée des parois 36 pour faciliter le transport.

La figure 6 montre les jointures entre les parois 38, 36 et 32 d'une part et la porte 34 d'autre part, jointures dans lesquelles sont disposées des dispositifs de fermeture étanche. La porte 34 pouvant être ouverte telle qu'illustrée, les deux lèvres de certaine jointure 50 de la porte peuvent être disjointes. La jointure 48 reliant la porte 34 et la paroi supérieure 38, la jointure 40 reliant la porte 34 et la paroi latérale 36 et la jointure 42 reliant la porte 34 et la paroi inférieure 32, peuvent ainsi être chacune disjointe en deux lèvres sur les parois 34 et 36 respectives que ces jointures elles relient. Le dispositif de fermeture étanche de chaque jointure peut être présent sur une des deux lèvres seulement de la jointure ou sur les deux lèvres de la jointure selon les modes de réalisation du dispositif de fermeture étanche.

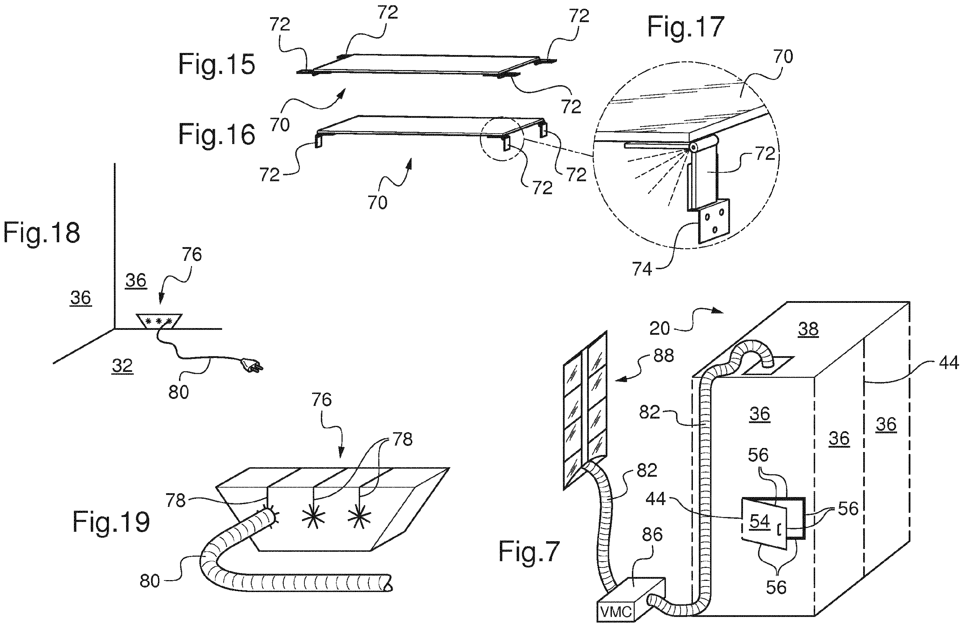

Selon un mode de réalisation, le dispositif de fermeture entre deux parois adjacentes dans la position d'utilisation peut être à fermeture magnétique. Le dispositif étanche à fermeture magnétique peut être formé par des bandes magnétiques enduites de silicone disposée sur chacune des deux lèvres d'une jointure. Les dispositifs étanches à fermeture magnétique sont de préférence utilisées pour la paroi formant la porte 34 ou tout autre paroi formant une ouverture amovible d'accès au volume 22 de la cabine 20 dans la position d'utilisation. La figure 7 montre une vue en perspective de l'arrière de la cabine 20. Telle qu'illustrée, la cabine 20 comporte de préférence une trappe 54 d'accès au volume de la cabine, ou passe plat. L'étanchéité de la trappe 54 avec les autres parois 30 de la cabine est de préférence réalisé par une articulation étanche à la jointure 44 et des dispositifs étanches à fermeture magnétique aux jointures 56.

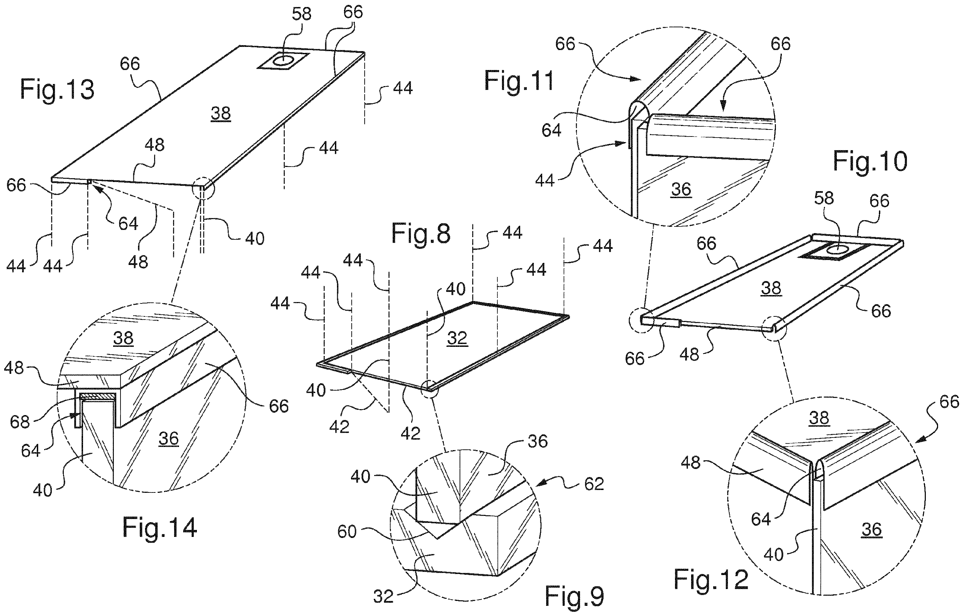

Selon un autre mode de réalisation, le dispositif de fermeture entre deux parois adjacentes 30 peut comporter une gorge. Ce mode de réalisation du dispositif de fermeture est préféré entre les parois adjacentes dont la jointure est comprimée dans position d'utilisation de la cabine 20 sous l'effet de la gravité. La figure 8 montre la paroi inférieure 32 sur laquelle le positionnement des parois latérales 34 et 36 est figuré par des traits discontinus au niveau de leurs jointures 40, 42 et 44. Selon le mode de réalisation illustrée la paroi 32 est un tapis d'étanchéité permettant de rattraper les décalages du sol du local dans lequel est disposée la cabine 20. La figure 9 montre une vue agrandie d'un détail de la figure 8 au niveau d'une jointure 62 de la paroi inférieure 32 et d'une des parois latérales 36. Cette jointure 62 comporte une gorge 60 en V en tant que dispositif de fermeture entre la paroi latérale 36 et la paroi inférieure 32. La gorge 60 en V facilite l'installation des parois 30 de la cabine qui glissent dans la gorge avec un maintien latéral dans la gorge. L'étanchéité de cette jointure 62 est réalisée par la compression de la gorge entre la paroi latérale 36 et la paroi inférieure 32 sous l'effet du poids de la paroi 36. De manière similaire, un dispositif de fermeture étanche sous forme de gorge 64 peut être disposé aux jointures 66 entre la paroi supérieure 38 et les parois latérales tel qu'illustré en figures 10, 11, et 12, les figure 11 et 12 montrant des vues agrandies de la figure 10 aux intersections entre les jointures 44 et 66 et les jointures 40, 48 et 66 respectivement. L'étanchéité aux jointures 66 est réalisée au niveau des gorges 64 par la compression entre la paroi supérieure 38 et les parois latérales 36 sous l'effet du poids de la paroi supérieure 38.

La figure 13 montre une vue en perspective de la paroi supérieure 38 sous laquelle le positionnement des parois latérales 34 et 36 est figuré par des traits discontinus au niveau de leurs jointures 40, 44 et 48, le dispositif de fermeture aux jointures 66 comprenant la gorge 64. De manière complémentaire au mode de réalisation comprenant une gorge ou en alternative, les dispositifs de fermeture étanche peuvent comprendre un joint plat en élastomère. La figure 14 montre une vue agrandie de la figure 13 à l'intersection des jointures 40, 48 et 66 avec un élastomère 68 dans la gorge 64 de la jointure 66. L'élastomère 68 est alors comprimé entre la paroi supérieure 38 et la paroi 36 sous l'effet du poids de la paroi 38 assurant ainsi une plus grande étanchéité au niveau de la jointure 66.

Tel qu'illustré en figures 4 et 5 et de manière générale, le matériau utilisé pour la réalisation des panneaux est de préférence un matériau alvéolaire pour limiter le poids total de la cabine 20. Ainsi selon un mode de réalisation proposé, le poids de la cabine 20 peut être inférieur ou égal à 25Kg voir inférieur ou égal à 23Kg. La réduction du poids de la cabine 20 permet de faciliter le transport et la manipulation de la cabine 20 par une personne seule tout en respectant des contraintes légales. Le matériau des parois 30 peut par ailleurs être choisi pour limiter le relargage d'odeur d'une expérience sensorielle précédente. Le matériau des parois peut ainsi être non poreux pour ne garder aucune mémoire des arômes. Compte tenu de l'ensemble des fonctions précédemment évoquées pour la cabine 20, le matériau choisi pour les parois est de préférence du PVC alvéolaire tel que de l'Aquilux. Les parois peuvent présenter une épaisseur de 8m ± 2mm, de préférence ± 1mm

Le volume fermé 22 de la cabine proposée 20 peut être adaptée à tous types d'analyses ou tests sensoriels. La cabine 20 peut ainsi comprendre une tablette amovible permettant la disposition d'équipement spécifique pour l'analyse sensorielle à conduire : vasque, crachoir, négatoscope, système informatique. La figure 15 montre une tablette 70, précédemment illustrée en figure 2. La tablette 70 comporte des équerres articulées 72, qui peuvent être mis à plat telle qu'illustré en figure 15 ou en figure 2 dans la position de transport. La figure 16 montre la tablette avec les équerres 72 en position perpendiculaire prêtes à être installées dans la cabine en position d'utilisation. La figure 17 montre une vue agrandie d'une équerre 72 de la tablette 70 reçue dans un support 74. Les parois latérales 36 de la cabine 20 peuvent ainsi présenter de tels supports 74 pour permettre une accroche facilitée de la tablette 70 tout en assurant une compacité élevée de chaque paroi 36 dans la position de transport de la cabine 20.

Pour faciliter le raccordement d'équipements disposés à l'intérieur de la cabine 20 avec l'extérieur, il est en outre proposé un pain de mousse comportant des trous préformés. La figure 18 montre un tel pain de mousse sous la forme d'une cale trapézoïdale 76 disposée dans une ouverture correspondante d'une des parois latérales 36. La figure 19 montre une vue agrandie de la cale 76. Cette cale 76 est de préférence réalisée en mousse élastomère à forte mémoire pour permettre l'insertion étanche en compression dans l'ouverture correspondante de la paroi 36. Le passage étanche des fils ou tuyaux 80 au travers de la cale 76, et donc de la cabine 20, se fait au niveau de découpes étoilés 78 s'adaptant à de multiples éléments de tailles, de formes et de sections différentes, tout en préservant l'étanchéité de la cabine 20.

Différents moyens ont été décrits pour réaliser l'étanchéité du volume fermé de la cabine 20 dans la position d'utilisation. La cabine 20 assure ainsi une atmosphère désaromatisée, nécessaire à la préservation d'un cadre expérimental olfactif toujours identique et ce avec une mobilité permise par le caractère pliable de la cabine.

Toutefois, la cabine 20 fermée et dans la position d'utilisation peut aussi permettre une circulation d'air entre l'extérieur et l'intérieur de la cabine 20. Cette circulation d'air entre l'intérieur et l'extérieur de la cabine 20 est de préférence contrôlée pour assurer une qualité d'air à l'intérieur de la cabine permettant la réalisation de l'analyse sensorielle. Ainsi une ou plusieurs parois 30 peuvent être adaptée à filtrer la circulation d'air traversant la paroi 30. Selon le mode de réalisation illustré en figure 6, la porte 34 comporte ainsi une cartouche de filtration 90 comportant, par exemple du charbon actif mélangé à des fibres, adapté à filtrer l'air circulant au travers de la porte 34. La porte peut comporter une grille mobile 84 d'accès à la cartouche dans la porte 34 pour faciliter la maintenance du filtre. La filtration proposée de la circulation d'air entre l'intérieur et l'extérieur de la cabine 20 est particulièrement utile pour le renouvellement en air de l'intérieur de la cabine 20 tout en limitant l'introduction d'odeurs parasites à l'analyse sensorielle. Pour contribuer au renouvellement contrôlé de l'air de la cabine 20, la cabine 20 peut aussi être associée à un moyen de ventilation, par exemple pour l'extraction d'air de la cabine 20. Selon le mode de réalisation illustré en figure 1, la cabine 20 peut ainsi être raccordée à une gaine de ventilation 82 permettant l'extraction d'air de la cabine 20. Cette gaine de ventilation 82 est par exemple reliée à une ventilation mécanique contrôlée 86, plus connus sous l'abréviation VMC illustrée en figure 7. L'association de la VMC 86 et de la cabine 20 forme un système d'analyse sensorielle également proposée. L'air extrait par la VMC 86 peut être évacué par toute ouverture du local dans lequel est disposés la cabine 20, telle qu'une fenêtre 88 ou une bouche d'aération (non illustrée) du local. Dans le cas où le local d'installation de la cabine 20 comporte une telle fenêtre, un tablier souple d'occultation de la fenêtre 88 peut être prévu afin de contrôler la luminosité ambiante à l'extérieur de la cabine 20. Le contrôle de la luminosité à l'extérieur de la cabine 20 peut être utile lorsque la cabine 20 présente des parois transparentes, telle que la vitre 24 de la porte 34, pour éviter une trop grande sensation d'enfermement de l'utilisateur dans la cabine 20.

Pour faciliter le raccordement entre la cabine 20 et la gaine de ventilation 82 de la VMC 86, la cabine 20 peut comporter une ouverture adaptée à être reliée de manière étanche à la gaine de ventilation 82. Telle qu'illustrée en figures 10 et 13, cette ouverture 58 peut être ménagée dans la paroi supérieure 38. Pour assurer une étanchéité au raccordement de la gaine 82 et de la cabine 20, l'ouverture 58 peut être pourvue d'un dispositif de fermeture magnétique, telle que précédemment décrit pour les jointures 40, 42 et 48.

Bien entendu, la présente invention n'est pas limitée aux exemples et au mode de réalisation décrit et représenté, mais elle est susceptible de nombreuses variantes accessibles à l'homme de l'art, selon les revendications suivantes. La porte 34 illustrée en figure 6 peut notamment comprendre une roue de contreventement 26 limitant les déformations de la porte 34 lors de son déplacement entre une position ouverte et fermée.

## Revendications

1. Cabine d'isolation sensorielle pliable entre
- une position repliée de transport et
- une position dépliée d'utilisation formant un volume fermé d'accueil (22) d'un utilisateur pour réaliser une analyse sensorielle ;
la cabine (20) comportant :
- des parois (30 : 32, 34, 36, 38), chaque paroi (30 : 32, 34, 36, 38) étant étanche à l'air ou adaptée à filtrer la circulation d'air traversant la paroi ;
- des articulations et des dispositifs de fermeture étanches à l'air, disposés à la jointure (40, 42, 44, 48, 62, 66) des parois (30 : 32, 34, 36, 38),
les articulations et les dispositifs de fermetures fermés reliant les parois (30 : 32, 34, 36, 38) de manière étanche à l'air dans la position dépliée d'utilisation de la cabine (20),
les articulations repliées autorisant la superposition des parois (30 : 32, 34, 36, 38) les unes sur les autres dans la position de transport de la cabine (20), carctérisée en ce que les parois (30 : 32, 34, 36, 38) comprennent une première et une deuxième parois (34, 36) réalisées en un même matériau commun, la première et la deuxième parois (34, 36) étant reliées entre elles de manière étanche par le matériau commun qui est aminci localement pour former une des articulations.

2. Cabine selon la revendication 1, dans laquelle les dimensions et le nombre de parois (30 : 32, 34, 36, 38) sont choisis de sorte que le volume fermé d'accueil (22) dans la position d'utilisation soit supérieur ou égal à 1,9*1*1 = 1.5 m³ de préférence supérieur ou égale à 1,9m³ et que le volume d'une enveloppe externe parallélépipédique de la cabine (20) dans la position de transport soit inférieur ou égal à 0.5 m³ de préférence inférieur ou égal à 1*1.9*0.1 = 0.2m³.

3. Cabine selon la revendication 2, dans laquelle l'enveloppe externe parallélépipédique de la cabine (20) dans la position de transport présente une longueur inférieure ou égale à 2.5m, de préférence inférieure ou égale à 2m, une largeur inférieure ou égale à 1.5m, de préférence inférieure ou égale à 1.1m et une épaisseur inférieure ou égale à 0.3m, de préférence inférieure ou égale à 0.1m.

4. Cabine selon l'une quelconque des revendications 1 à 3, le poids de la cabine (20) étant inférieur ou égale à 25Kg, de préférence inférieur ou égale à 23Kg.

5. Cabine selon l'une quelconque des revendications 1 à 4, dans laquelle les parois (30 : 32, 34, 36, 38) sont réalisées en matériau alvéolaire, de préférence en PVC alvéolaire.

6. Cabine selon l'une quelconque des revendications 1 à 5, dans laquelle les parois (30 : 32, 34, 36, 38) comprennent une paroi formant une porte (34) ou une trappe (54) d'accès au volume fermé d'accueil (22), la porte (34) ou la trappe (54) comportant un dispositif de fermeture étanche à l'air.

7. Cabine selon la revendication 6, dans laquelle le dispositif de fermeture étanche à l'air est à fermeture magnétique.

8. Cabine selon l'une quelconque des revendications 1 à 7, les parois comportent :
- au moins une paroi horizontale (32, 38) comprenant une paroi supérieure (38) et de préférence une paroi inférieure (32) ;
- des parois latérales (34, 36) ;
les dispositifs de fermeture étanche de la cabine (20) comprenant, à la jointure (62, 66) de l'au moins une paroi horizontale (32, 38) et des parois latérales (34, 36) dans la position dépliée d'utilisation de la cabine (20), un dispositif de fermeture étanche comportant au moins un parmi un joint élastomère (68) et une gorge (60, 64), l'étanchéité à la jointure de l'au moins une paroi horizontale (32, 38) et des parois latérales (34, 36) étant réalisée par la compression du dispositif de fermeture étanche entre les parois latérales (34, 36) et l'au moins une paroi horizontale (32, 38) sous l'effet du poids des parois (32, 34, 36, 38).

9. Cabine selon l'une quelconque des revendications 1 à 8, dans laquelle les parois (30 : 32, 34, 36, 38) comportent une paroi (34) muni d'une cartouche (90) de filtration adaptée à filtrer l'air circulant au travers de la paroi (34).

10. Système d'analyse sensorielle comprenant :
- une ventilation mécanique contrôlée (86) ;
- la cabine (20) selon l'une des revendications 1 à 10, la cabine (20) comportant une ouverture (58) adaptée à être reliée de manière étanche à la ventilation mécanique contrôlée (86) pour le renouvellement de l'air du volume fermé d'accueil (22) dans la position d'utilisation.

## Patentansprüche

1. Sensorische-isolierungs Kabine die faltbar ist zwischen
- einem zusammengeklappten Transportzustand und
- einem aufgeklappten Verwendungszustand, wobei ein abgeschlossenes Volumen zur Aufnahme (22) eines Anwenders zur Durchführung einer sensorischen Analyse gebildet wird;
welche Kabine (20) umfasst:
- Wände (30: 32, 34, 36, 38), wobei jede Wand (30: 32, 34, 36, 38) luftdicht oder ausgelegt ist, den durch die Wand gehenden Luftstrom zu filtern;
- luftdichte Gelenke und Verschlussvorrichtungen, die an der Verbindungsstelle (40, 42, 44, 48, 62, 66) der Wände (30: 32, 34, 36, 38) angeordnet sind,
wobei
o die Gelenke und die geschlossenen Verschlussvorrichtungen im aufgeklappten Verwendungszustand die Wände (30: 32, 34, 36, 38) der Kabine (20) luftdicht verbinden,
o die zusammengeklappten Gelenke das gegenseitige Stapeln der Wände (30: 32, 34, 36, 38) im Transportzustand der Kabine (20) ermöglichen,
**dadurch gekennzeichnet, dass** die Wände (30: 32, 34, 36, 38) eine erste und eine zweite Wand (34, 36) umfassen, die aus demselben gemeinsamen Material hergestellt sind, wobei die erste und die zweite Wand (34, 36) durch das gemeinsame Material, das zur Bildung eines der Gelenke lokal verdünnt ist, miteinander dicht verbunden sind.

2. Kabine nach Anspruch 1, wobei Größe und Anzahl der Wände (30: 32, 34, 36, 38) so ausgewählt werden, dass das abgeschlossene Volumen zur Aufnahme (22) im Verwendungszustand größer oder gleich 1,9*1*1 = 1,5 m³, bevorzugt größer oder gleich 1,9 m³, und das Volumen einer quaderförmigen Außenhülle der Kabine (20) im Transportzustand kleiner oder gleich 0,5 m³, bevorzugt kleiner oder gleich 1*1,9*0,1 = 0,2 m³, ist.

3. Kabine nach Anspruch 2, wobei die quaderförmige Außenhülle der Kabine (20) im Transportzustand eine Länge, die kleiner oder gleich 2,5 m ist, bevorzugt kleiner oder gleich 2 m, eine Breite, die kleiner oder gleich 1,5 m ist, bevorzugt kleiner oder gleich 1,1m, und eine Dicke, die kleiner oder gleich 0,3 m ist, bevorzugt kleiner oder gleich 0,1 m, aufweist.

4. Kabine nach einem der Ansprüche 1 bis 3, wobei das Gewicht der Kabine (20) kleiner oder gleich 25 kg ist, bevorzugt kleiner oder gleich 23 kg.

5. Kabine nach einem der Ansprüche 1 bis 4, wobei die Wände (30: 32, 34, 36, 38) aus einem Schaumstoffmaterial hergestellt sind, bevorzugt aus PVC-Schaumstoff.

6. Kabine nach einem der Ansprüche 1 bis 5, wobei die Wände (30: 32, 34, 36, 38) eine Wand umfassen, die eine Tür (34) oder eine Klappe (54) als Zugang zu dem abgeschlossenen Volumen zur Aufnahme (22) bildet, wobei die Tür (34) oder die Klappe (54) eine luftdichte Verschlussvorrichtung umfasst.

7. Kabine nach Anspruch 6, wobei die luftdichte Verschlussvorrichtung ein Magnetverschluss ist.

8. Kabine nach einem der Ansprüche 1 bis 7, wobei die Wände aufweisen:
- mindestens eine horizontale Wand (32, 38), umfassend eine obere Wand (38) und bevorzugt eine untere Wand (32);
- Seitenwände (34, 36);
wobei die dichten Verschlussvorrichtungen der Kabine (20), im aufgeklappten Verwendungszustand der Kabine (20), an der Verbindungsstelle (62, 66) der mindestens einen horizontalen Wand (32, 38) und der Seitenwände (34, 36), umfasst eine dichte Verschlussvorrichtung, die zumindest eine elastomere Dichtung (68) und eine Nut (60, 64) aufweist, wobei die Dichtigkeit an der Verbindungsstelle der mindestens einen horizontalen Wand (32, 38) und der Seitenwände (34, 36) durch das Komprimieren der dichten Verschlussvorrichtung zwischen den Seitenwänden (34, 36) und der mindestens einen horizontalen Wand (32, 38) unter der Wirkung des Gewichtes der Wände (32, 34, 36, 38) realisiert wird.

9. Kabine nach einem der Ansprüche 1 bis 8, wobei die Wände (30: 32, 34, 36, 38) eine Wand (34) mit einer Filterkartusche (90) umfasst, die geeignet ist, die durch die Wand (34) strömende Luft zu filtern.

10. Sensorisches Analysesystem, umfassend:
- eine mechanisch geregelte Belüftung (86);
- die Kabine (20) nach einem der Ansprüche 1 bis 9, wobei die Kabine (20) eine Öffnung (58) aufweist, die mit der mechanisch geregelten Belüftung (86) dicht verbindbar ist, um die Luft des abgeschlossenen Volumens zur Aufnahme (22) im Verwendungszustand auszutauschen.

## Claims

1. Sensory isolation booth that is foldable between
- a folded transport position and
- an unfolded position of use forming a closed volume for receiving (22) a user to perform a sensory analysis;
the booth (20) comprising:
- walls (30 : 32, 34, 36, 38), each wall (30 : 32, 34, 36, 38) being airtight or suitable for filtering the circulation of air through the wall;
- airtight articulations and closing devices positioned at the join (40, 42, 44, 48, 62, 66) of the walls (30 : 32, 34, 36, 38),
the articulations and closing devices, when closed, connecting the walls (30 : 32, 34, 36, 38) in an airtight manner in the unfolded position of use of the booth (20), the folded articulations allowing the superposition of the walls (30 : 32, 34, 36, 38) on one another in the transport position of the booth (20), **characterized in that** the walls (30 : 32, 34, 36, 38) comprise a first and a second wall (34, 36) made of the same common material, the first and the second walls (34, 36) being connected to each other in an airtight manner by the common material which is thinned locally in order to form one of the articulations.

2. Booth according to claim 1 in which the dimensions and the number of walls (30 : 32, 34, 36, 38) are chosen so that the closed reception volume in the position of use is greater than or equal to 1,9*1*1 = 1.5 m³, preferably greater than or equal to 1.9 m³, and the volume of an external parallelepiped cover for the booth (20) in the transport position is less than or equal to 0.5 m³, preferably less than or equal to 1*1.9*0.1 = 0.2m³.

3. Booth according to claim 2, in which the external parallelepiped cover for the booth (20) in the transport position has a length less than or equal to 2.5 m, preferably less than or equal to 2m, a width less than or equal to 1.5 m, , preferably less than or equal to 1.1m and a depth less than or equal to 0.3 m, , preferably less than or equal to 0.1m.

4. Booth according to one of claims 1 to 3, the weight of the booth (20) being less than or equal to 25 kg, , preferably less than or equal to 23 kg.

5. Booth according to one of claims 1 to 4, in which the walls (30 : 32, 34, 36, 38) are made of a cellular material, preferably a cellular PVC.

6. Booth according to one of claims 1 to 5, in which the walls (30 : 32, 34, 36, 38) comprise a wall forming a door (34) or an access hatch (54) to the closed reception volume (22), the door (34) or the hatch (54) comprising an airtight closing device.

7. Booth according to claim 6, in which the airtight closing device closes magnetically.

8. Booth according to one of claims 1 to 7, in which the walls comprise:
- at least one horizontal wall (32, 38) comprising an upper wall (38) and preferably a lower wall (32);
- side walls (34, 36);
the airtight closing devices of the booth (20) comprising, at the join (62, 66) of the at least one horizontal wall (32, 38) and the side walls (34, 36) in the unfolded position of use of the booth (20), an airtight closing device comprising at least one elastomer seal (68) and a groove (60, 64) the airtightness at the join of the at least one horizontal wall (32, 38) and the side walls (34, 36) being produced by compression of the airtight closing device between the side walls (34, 36) and the at least one horizontal wall (32, 38) under the effect of the weight of the walls (32, 34, 36, 38).

9. Booth according to one of claims 1 to 8, in which the walls (30 : 32, 34, 36, 38) comprise a wall (34) equipped with a filter cartridge (90) suitable for filtering the air circulating through the said wall (34).

10. System for sensory analysis comprising:
- a controlled mechanical ventilation system (86);
- the booth (20) according to one of claims 1 to 9, the booth (20) comprising an opening (58) suitable for being connected in an airtight manner to the controlled mechanical ventilation system (86) in order to change the air in the closed reception volume (22) in the position of use.
